Europäisches Patentamt

(19) European Patent Office

Office européen des brevets

(11) Numéro de publication : **0 237 538 B1**

(12) # FASCICULE DE BREVET EUROPEEN

(45) Date de publication du fascicule du brevet :
21.08.91 Bulletin 91/34

(51) Int. Cl.⁵ : **A61K 9/54, A61K 31/165**

(21) Numéro de dépôt : **86905319.9**

(22) Date de dépôt : **12.09.86**

(86) Numéro de dépôt international :
**PCT/FR86/00306**

(87) Numéro de publication internationale :
**WO 87/01588 26.03.87 Gazette 87/07**

---

(54) PREPARATION D'UN MEDICAMENT RETARD.

(30) Priorité : **12.09.85 JP 202855/85**

(43) Date de publication de la demande :
**23.09.87 Bulletin 87/39**

(45) Mention de la délivrance du brevet :
**21.08.91 Bulletin 91/34**

(84) Etats contractants désignés :
**AT BE CH DE FR GB IT LI LU NL SE**

(56) Documents cités :
**EP-A- 0 092 060**
**EP-A- 0 148 811**
**WO-A-82/01468**
**DE-B- 1 949 894**
**See also references of WO8701588**

(73) Titulaire : **SOCIETE D'ETUDES**
**SCIENTIFIQUES ET INDUSTRIELLES DE**
**L'ILE-DE-FRANCE**
**46, Boulevard de Latour-Maubourg**
**F-75340 Paris Cédex 07 (FR)**

(72) Inventeur : **HATA, Takehisa**
**13-7, Nishikakiuchi Terado-cho**
**Muko-shi Kyoto (JP)**
Inventeur : **YAMAGUCHI, Hisami**
**17-12-705, Mondoso Nishinomiya-shi**
**Hyogo (JP)**
Inventeur : **UEDA, Satoshi**
**246-16, Nitta Kawanishi-shi**
**Hyogo (JP)**
Inventeur : **KODANI, Masateru**
**8-6-13, Minoo Minoo-shi**
**Osaka (JP)**

(74) Mandataire : **Gallochat, Alain**
**SOCIETE D'ETUDES SCIENTIFIQUES ET**
**INDUSTRIELLES DE L'ILE DE FRANCE 46,**
**Boulevard de Latour Maubourg**
**F-75340 Paris Cédex 07 (FR)**

## Description

La présente invention concerne une préparation retard à base de métoclopramide.

D'après le brevet EP-A-92060, on connaissait des compositions pharmaceutiques à libération prolongée du principe actif, constituées d'une matrice formée d'un polymère, dans laquelle sont incorporés le principe actif et des excipients, recouverte d'une première couche constituée d'un polymère soluble dans l'eau et d'une deuxième couche protectrice, constituée d'un polymère insoluble dans l'eau.

D'après le brevet EP-A-148811, on connaissait des compositions pharmaceutiques à diffusion améliorée du principe actif dans la partie inférieure du système gastrointestinal, comportant un noyau contenant le principe actif, recouvert d'une membrane de diffusion constituée d'éthylcellulose et/ou d'un copolymère acrylique, et d'une couche externe composée d'un ou plusieurs polymères anioniques et/ou d'un acide gras.

D'après le brevet WO-A-82/1468, on connaissait une composition pharmaceutique à libération prolongée du métoclopramide, comportant un noyau neutre recouvert de couches successives de métoclopramide et d'excipients et d'une couche externe microporeuse constituée d'au moins un polymère.

La préparation selon la présente invention est caractérisée par l'enrobage d'un noyau comprenant le métoclopramide, d'abord avec une substance qui en retarde la délivrance : un copolymère de méthacrylate de méthyle et d'acrylate d'éthyle (connu sous le nom de EUDRAGIT E30D), puis avec un revêtement entérique : le phtalate d'hydroxypropylméthyl cellulose (connu sous le nom de HP-55).

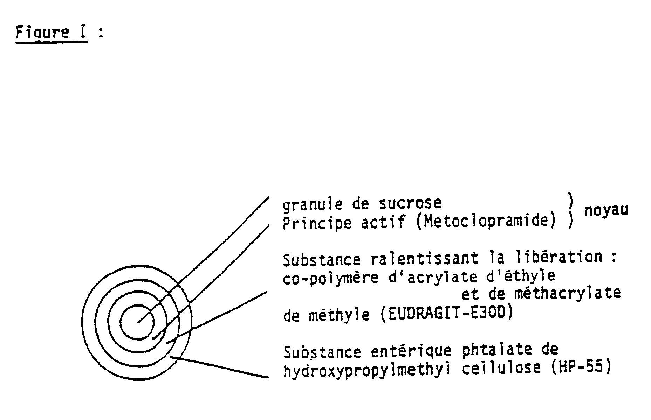

Le schéma de la figure I indique la structure de la préparation.

La composition selon l'invention est caractérisée en ce qu'elle assure une libération régulière du principe actif tout au long du tractus gastrointestinal.

Le principe actif peut être utilisé soit sous forme de granule, soit déposé autour d'une âme neutre telle qu'un granule de sucrose. Puis on procède à l'enrobage avec la substance apte à ralentir la libération du principe actif dans des conditions de dissolution acides ou basiques, de façon à assurer une dissolution constante du principe actif dans le tractus gastrointestinal.

Un exemple de réalisation de l'invention est donné afin d'illustrer la préparation.

## Exemple

Dans un granulateur centrifuge, on enrobe 400 parties de granules de sucrose (tamis mailles 32 à 42) d'une solution de résine SHELLAC (44 parties) dissoute dans de l'éthanol (290 parties) et mélangée à du talc (60 parties).

On revêt les granules de métoclopramide à l'aide d'une solution aqueuse à 5% de TC-5R (liant préparé par SHIN-ETSU Chemical) dans le granulateur centrifuge (le métoclopramide représentant 30% en poids du noyau). On enrobe ensuite les granules obtenus, contenant les 30% de métoclopramide, d'EUDRAGIT - E30D dans un granulateur à lit fluidisé. Une solution de HP-55 (15 g) dans un mélange 50-50 d'éthanol et de dichloroéthane (400 g) est pulvérisée sur les granules, dans le granulateur à lit fluidisé, pour obtenir la préparation retard de métoclopramide.

## Test de dissolution

### 1) Préparation du test :

L'échantillon (I) a été préparé selonn l'exemple ci-dessus. l'échantillon (II) a été préparé comme ci-dessus a l'exception de l'enrobage du HP-55.

### 2) Methode du test :

A) Pharmacopée japonaise (10e édition) dissolution : méthode II (barbottage) milieu de dissolution : 1er fluide (pH 1,2), 900 ml, 37°C, 100 r.p.m

B) Pharmacopée japonaise (10e édition) dissolution : méthode 11 (barbottage) milieu de dissolution : 2e fluide (pH 6,8), 900 ml, 37°C, 100 r.p.m

3) Résultat du test :

méthode A

|  | TEMPS (HEURES) | | | | | | | |
|---|---|---|---|---|---|---|---|---|
|  | 0.5 | 1 | 2 | 3 | 4 | 6 | 7 | 8 |
| Taux de dissolution (%) de l'échantillon (I) | - | 15 | 30 | 40 | 50 | 60 | 65 | 70 |
| Taux de dissolution (%) de l'échantillon (II) | 65 | 80 | 85 | 90 | 92 | 95 | 97 | 98 |

méthode B

|  | TEMPS HEURES | | | | | |
|---|---|---|---|---|---|---|
|  | 1 | 2 | 3 | 4 | 6 | 8 |
| Taux de dissolution (%) de l'échantillon (I) | 32 | 55 | 63 | 72 | 82 | 86 |
| Taux de dissolution (%) de l'échantillon (II) | 30 | 53 | 64 | 70 | 80 | 85 |

**Revendications**

**Revendication pour les Etats contractants suivants :BE, CH, DE, FR, GB, IT, LI, LU, NL, SE**

1. Composition pharmaceutique à diffusion contrôlée du métoclopramide, caractérisée en ce qu'elle comprend un noyau contenant du métoclopramide, puis une couche constituée d'un copolymère de méthacrylate de méthyle et d'acrylate d'ethyle et enfin un enrobage externe constitué de phtalate d'hydroxypropylméthyl cellulose.

**Revendication pour l'Etat contractant suivant : AT**

1. Procédé de préparation d'une composition pharmaceutique à diffusion contrôlée du métoclopramide, caractérisé en ce qu'un noyau contenant le métoclopramide est recouvert d'une couche constituée d'un copolymère de méthacrylate de méthyle et d'acrylate d'éthyle puis d'un enrobage constitué de phtalate d'hydroxypropylméthyl cellulose.

3

**Patentansprüche**

**Patentanspruch Für folgenden Vertragsstaaten : BE, CH, DE, FR, GB, IT, LI, LU, NL, SE**

1. Pharmazeutische Zusammensetzung mit kontrollierter Diffusion von Metoclopramid, dadurch gekennzeichnet, daß sie einen Metoclopramid enthaltenden Kern, weiterhin einen aus einem Methylmethacrylat-Ethylacrylat-Copolymeren bestehenden Überzug und schließlich eine äußere aus Hydroxypropylmethyl-cellulose-phthalat bestehende Umhüllung umfaßt.

**Patentanspruch Für folgenden Vertragsstaat : AT**

1. Verfahren zur Herstellung einer pharmazeutischen Zusammensetzung zur kontrollierten Diffusion von Metoclopramid, dadurch gekennzeichnet, daß ein Metoclopramid enthaltender Kern mit einem aus einem Methylmethacrylat-Ethylacrylat-Copolymeren bestehenden Überzug und anschließend mit einer aus Hydroxypropylmethyl-cellulose-phthalat bestehenden Umhüllung überzogen wird.

**Claims**

**Claim for the following Contracting States : BE, CH, DE, FR, GB, IT, LI, LU, NL, SE**

1. A pharmaceutical composition giving controlled diffusion of metoclopramide, characterised in that it comprises a core containing metoclopramide, then a layer made up of a copolymer of methyl methacrylate and ethyl acrylate, and finally an external coating made up of hydroxypropylmethyl cellulose phthalate.

**Claim for the following Contracting State : AT**

1. A method of preparing a pharmaceutical composition giving controlled diffusion of metoclopramide, characterised in that a core containing metoclopramide is covered with a layer made up of a copolymer of methyl methacrylate and ethyl acrylate, then a coating made up of hydroxypropylmethyl cellulose phthalate.

Figure I :

granule de sucrose              )
Principe actif (Metoclopramide) ) noyau

Substance ralentissant la libération :
co-polymère d'acrylate d'éthyle
                   et de méthacrylate
de méthyle (EUDRAGIT-E30D)

Substance entérique phtalate de
hydroxypropylmethyl cellulose (HP-55)